# EUROPEAN PATENT SPECIFICATION

(11) **EP 1 841 811 B1**
(45) Date of publication and mention of the grant of the patent: **02.05.2018**
(21) Application number: 05817904.5
(22) Date of filing: 20.12.2005
(51) Int. Cl.: C08J 3/12, C08J 3/205, C08L 29/04, A61K 9/16

(54) **IMPROVEMENTS RELATING TO RAPIDLY DISSOLVING COMPOSITIONS**
VERBESSERUNGEN BEZÜGLICH SICH SCHNELL AUFLÖSENDER ZUSAMMENSETZUNGEN
AMELIORATIONS APPORTEES A DES COMPOSITIONS DE DISSOLUTION RAPIDE

(30) Priority: 28.01.2005 GB 0501835
(43) Date of publication of application: 10.10.2007
(73) Proprietor: The University of Liverpool, Liverpool, L69 7ZX (GB)
(72) Inventor: BARNWELL, Stephen G Unilever R & D Port Sunlight, Wirral Merseyside CH63 3JW (GB); COOPER, Andrew, Ian University of Liverpool, Liverpool Merseyside CH69 3BX (GB); DUNCALF, David Unilever R & D Port Sunlight, Wirral Merseyside CH63 3JW (GB); FOSTER, Alison, Jayne Unilever R & D Port Sunlight, Wirral Merseyside CH63 3JW (GB); RANNARD, Stephen Paul Unilever R & D Port Sunlight, Wirral Merseyside CH 63 3JW (GB)
(74) Representative: Dauncey, Mark Peter
(86) International application number: PCT/EP2005/013933
(87) International publication number: WO 2006/079409

(56) References cited:
- DE-A1- 2 122 662
- US-A- 3 998 753
- US-A1- 2004 121 003

## Description

### Technical Field:

The present invention relates to dried compositions which dissolve rapidly in water.

### Background to the Invention:

Many useful materials are water-insoluble. These include, without limitation, many dyestuffs (i.e. pigments), some fluorescer materials and many pharmaceutical materials. In some circumstances it is possible to disperse these materials in water by processes involving fine comminution, but this can be expensive and time-consuming.

Our co-pending international patent application PCT/GB03/03226 describes the formation of solid, porous beads comprising a three dimensional open-cell lattice of a water-soluble polymeric material. These are typically 'templated' materials formed by the removal of both water and a non-aqueous dispersed phase from a high internal phase emulsion (HIPE) which has a polymer dissolved in the aqueous phase. The beads are formed by dropping the HIPE emulsion into a low temperature fluid such as liquid nitrogen, then freeze-drying the particles formed to remove the bulk of the aqueous phase and the dispersed phase. This leaves behind the polymer in the form of a 'skeletal' structure. DE 21 22 662 relates to a method for preparing water-dispersible dyes comprising the steps of dissolving water-insoluble dyes in powder form (A) in an water-immiscible organic solvent (B); dispersing the solution in water comprising a dispersing agent (C) preferably an anionic water-soluble salt) and optionally other dispersing, emulsifying or wetting agents (D); converting the mixture into an oil-in-water emulsion and spray-drying to remove the volatile constituents. The beads dissolve rapidly in water and have the remarkable property that a water-insoluble component dispersed in the dispersed phase of the emulsion prior to freezing and drying can also be dispersed in water on solution of the polymer skeleton of the beads.

The production process for the beads and related materials is complex. It requires the use of liquid gasses and vacuum equipment. Throughput is relatively low. There is a clear need to devise a simpler and cheaper process.

Past workers have looked at ways of delivering water-insoluble materials such as carotenoids. US 3998753 (Antoshkiw et al., 1974) discloses a method of preparing a water-dispersible carotenoid composition which comprises the steps of forming an emulsion of the carotenoid in a volatile solvent (such as chloroform) and various structurants (such as gelatine) in water. This emulsion is heated with high-speed mixing to drive off the volatile solvent and the resulting emulsion product can be used as is. Optionally, the solvent-free product may be further processed by spray drying to remove the water and give a dry product.

US 4213900 (Daubach et al., 1978) takes a different approach. Here formic acid, formamide, N-methylformamide, butyrolactone, ethylene or propylene glycols are used as solubilising agents for water-insoluble dyes. Once a solution has been formed it can be spray-dried to form a fine powder which can be dispersed in water.

Pamujula et al. (Journal of Pharmacy and Pharmacology, 2004, 56: 1119-1125) disclose a process for the production of spray dried poly(lactide-co-glycolide) (PLGA) carrier particles containing the cytoprotective drug amifostine. In the procedure described the drug was dissolved in water and emulsified with a solution of PLGA carrier in a water-immiscible solvent (dichloromethane). This was spray-dried to remove the majority of the solvent and obtain nanoparticles, which were then freeze-dried to remove any residual solvent. The final product of the process was in the form of nanoparticles having a median size of 240-257 nm.

Several other methods are known which result in a 'dry emulsion'. For example Dollo et al (Eur. J. of Pharmaceutical Sciences, 19, issue 4 pages 273-280) discloses a physically stabilised dry emulsion with a lipophilic drug dissolved in the oil phase.

Hansen, Holm and Schultz (Int J. Pharmaceutics, 287, issue 1-2, 9th December 2004, pages 55-66) disclose a process for preparing powders containing a poorly water-soluble drug dissolved in (non-volatile) medium chain triglyceride, by spray drying o/w emulsions. The products of this process would still contain the 'solvent' for the drug, i.e. non-volatile medium chain triglyceride. Thus the product of the spray drying process can be described as a 'dry emulsion' which will reform the initial emulsion on addition of water.

### Brief Description of the Invention:

We have now determined that spray drying can be used instead of freeze drying to obtain solid, rapidly dissolving materials directly from an emulsion.

Accordingly a first aspect of the present invention provides a method comprising the steps of:
(i) providing an emulsion of:
   a) an aqueous solvent,
   b) a carrier material soluble in (a), said carrier material being solid at ambient temperature,
   c) a volatile second liquid phase which is not miscible with (a), and
   d) a material which is soluble in (c) but not in (a), and,
(ii) spray-drying the emulsion at above 100 Celsius to simultaneously remove (a) and (c) and thereby obtain a powder, in which the carrier material (b) has material (d) dispersed therein, said dispersion of (d) being a nano-scale dispersion,
wherein the carrier material (b) is a polymer and/or a surfactant,
wherein the polymer is a water-soluble polymer selected from the group consisting of:
natural polymers;
cellulose derivatives;
homopolymers of any one of, or copolymers prepared from two or more of, the following monomers: vinyl alcohol, acrylic acid, methacrylic acid, acrylamide, methacrylamide, acrylamide methylpropane sulphonates, aminoalkylacrylates, aminoalkylmethacrylates, hydroxyethylacrylate, hydroxyethylmethylacrylate, vinyl pyrrolidone, vinyl imidazole, vinyl amines, vinyl pyridine, ethyleneglycol and other alkylene glycols, ethylene oxide and other alkylene oxides, ethyleneimine, styrenesulphonates, ethyleneglycolacrylates, ethyleneglycol methacrylate; and
mixtures thereof,
wherein the surfactant is non-ionic and/or anionic,
wherein the non-ionic surfactant is selected from the group consisting of:
ethoxylated triglycerides, fatty alcohol ethoxylates, alkylphenol ethoxylates, fatty acid ethoxylates, fatty amide ethoxylates, fatty amine ethoxylates, sorbitan alkanoates, ethylated sorbitan alkanoates, alkyl ethoxylates, Pluronics™, alkyl polyglucosides, stearol ethoxylates, alkyl polyglycosides, and mixtures thereof, and
wherein the anionic surfactant is selected from the group consisting of:
alkylether sulfates, alkylether carboxylates, alkylbenzene sulfonates, alkylether phosphates, dialkyl sulfosuccinates, alkyl sulfonates, soaps, alkyl sulfates, alkyl carboxylates, alkyl phosphates, paraffin sulfonates, secondary n-alkane sulfonates, alpha-olefin sulfonates, isethionate sulfonates, and mixtures thereof.

The aqueous solvent (a) is generally and preferably water, but may comprise an electrolyte solution or a mixture of hydrophilic solvents.

Both the aqueous solvent (a) and the volatile, water-immiscible second liquid phase (c) are simultaneously removed during the spray-drying process, and no separate removal step for either the second liquid phase (e.g. by boiling it off) or the aqueous solvent (e.g. by freeze drying) is needed. By 'volatile' here is meant that the second liquid phase is sufficiently volatile to be removed as a vapour phase during the drying process. 'Burning-off' of one of the solvents is not required in the method of the invention.

Spray-drying is particularly effective at removing both the aqueous and non-aqueous volatile components to leave the carrier and the 'payload' material behind in a fine particulate form.

Drying is conducted at a temperature above 100 Celsius. In the case of a spray drying apparatus this means that the inlet temperature of the spray drying apparatus is, for example, above 100 Celsius. Particularly preferred drying conditions are those in which the drying temperature is above both the boiling point of water and the boiling point of the volatile second liquid phase.

The final structure of the material obtained is not well understood. However it is believed that removal by spray-drying of the aqueous solvent and the second liquid phase results in a very fine, nano-scale
dispersion of the water insoluble material (d) through the water-soluble carrier material (b). It is believed that the resulting dry materials are not encapsulates, as discrete bodies of the water-immiscible materials (d) are not present in the dry product. Neither are the dry materials 'dry emulsions' as little or none of the volatile solvent comprising the 'oil' phase of the emulsion remains after the drying step. On addition of water to the dry product the emulsion is not reformed, as it would be with a 'dry emulsion'.

On admixture of the carrier material with water, the carrier (b) dissolves and the water insoluble material (d) is dispersed through the water in sufficiently fine form that it behaves like a soluble material in many respects. Surprisingly, the effectiveness of materials delivered in this way is hardly reduced as compared with materials delivered using an emulsion- templated, freeze-drying preparation method.

### Detailed Description of the Invention:

In order that the present invention may be better understood and carried forth into practice, it is described below with reference to various preferred features and particular embodiments.

### Carrier Material:

The carrier material is water soluble, which includes the formation of structured aqueous phases as well as true ionic solution of molecularly mono-disperse species. The carrier material is a surfactant, a polymer or a mixture of two or more of these.

### Polymeric Carrier Materials:

When present, the polymeric carrier material is a material that would be considered as "water soluble" by those skilled in the art, i.e. if it forms a homogeneous solution in water. Water soluble polymers generally possess pendant polar or ionizable groups (e.g. -C=O, -OH, -N(R₁)(R₂) in which R₁ and R₂, which may be the same or different, are independently H or (C1 to C4)alkyl, -N(R₃)(R₄)(R₅)⁺ in which R₃, R₄ and R₅, which may be the same or different, are independently H or (C1 to C4)alkyl, -CON(R₆)(R7) in which R₆ and R₇, which may be the same or different, are H or (C1 to C4)alkyl, -CH₂CH₂O-, -CO₂H or salts thereof, -SO₃H or salts thereof) on a backbone chain which may be hydrophobic.

If water-soluble polymeric carrier materials are incorporated into the products of the present invention, the time taken for the products to dissolve may be significantly reduced, as compared with 'off-the-shelf' samples of the polymeric carrier materials. It is believed that this is due to the structural result of, in particular, the spray-drying process. The same is believed to be true of surfactants. The nature of the products should
be such that the dissolution of the products in a large excess of water preferably occurs in less than three minutes, more preferably less than two minutes, most preferably less than one minute.

The particle size and distributions of the 'payload' material can be tailored by choosing different water-soluble polymers and polymers with different molecular weights. This allows some control over the activity/availability of the 'payload material'.

Examples of suitable water-soluble polymeric carrier materials include:
(a) naturally occurring gums such as guar gum or locust bean gum, or a polysaccharide such as dextran, as natural polymers; and
(b) xanthan gum, xyloglucan, cellulose acetate, methylcellulose, methyl-ethylcellulose, hydroxyethylcellulose, hydroxyethylmethyl-cellulose, hydroxypropylcellulose, hydroxypropylmethylcellulose (HPMC), hydroxypropylbutylcellulose, ethylhydroxyethylcellulose, carboxymethylcellulose and its salts (e.g. the sodium salt - SCMC), or carboxymethylhydroxyethylcellulose and its salts (for example the sodium salt as cellulose derivatives.

When the polymeric material is a copolymer it may be a statistical copolymer (heretofore also known as a random
(d) copolymers prepared from two or more monomers listed in Table 1 below;
(e) mixtures thereof

### Table 1

vinyl alcohol,
acrylic acid,
methacrylic acid
acrylamide,
methacrylamide
acrylamide methylpropane sulphonates
aminoalkylacrylates
aminoalkylmethacrylates
hydroxyethylacrylate
hydroxyethylmethylacrylate
vinyl pyrrolidone
vinyl imidazole
vinyl amines
vinyl pyridine
ethyleneglycol and other alkylene glycols
ethylene oxide and other alkylene oxides
ethyleneimine
styrenesulphonates
ethyleneglycolacrylates
ethyleneglycol methacrylate

When the polymeric material is a copolymer it may be a statistical copolymer (heretofore also known as a random copolymer), a block copolymer, a graft copolymer or a hyperbranched copolymer. Comonomers other than those listed may also be included in addition to those listed if their presence does not destroy the water soluble nature of the resulting polymeric material.

Examples of suitable homopolymers include polyvinylalcohol, polyacrylic acid, polymethacrylic acid, polyacrylamides (such as poly-N-isopropylacrylamide), polymethacrylamide; polyacrylamines, polymethylacrylamines, (such as polydimethylaminoethylmethacrylate and poly-N-morpholinoethylmethacrylate, polyvinyl-pyrrolidone, polyvinylimidazole, polyvinylpyridine, polyethylene-imine and ethoxylated derivatives thereof. Polyvinylalcohol is a particularly preferred carrier material.

### Surfactant Carrier Materials:

Mixtures of surfactants may be used. In such mixtures there may be individual components which are liquid, provided that the carrier material overall, is a solid.

Anionic surfactants are particularly preferred as carrier materials.

### Second liquid phase:

The compositions of the invention comprise a volatile, second liquid phase, which is not miscible with the aqueous solvent.

The second liquid phase of the emulsion may be selected from one or more from the following group of volatile organic solvents:
- alkanes, such as heptane, n-hexane, isooctane, dodecane, decane;

Examples of suitable anionic surfactants include alkylether sulfates; alkylether carboxylates; alkylbenzene sulfonates; alkylether phosphates; dialkyl sulfosuccinates; alkyl sulfonates; soaps; alkyl sulfates; alkyl carboxylates; alkyl phosphates; paraffin sulfonates; secondary n-alkane sulfonates; alpha-olefin sulfonates; isethionate sulfonates.

Examples of suitable cationic surfactants include fatty amine salts; fatty diamine salts; quaternary ammonium compounds; phosphonium surfactants; sulfonium surfactants; sulfonxonium surfactants.

Examples of suitable zwitterionic surfactants include N-alkyl derivatives of amino acids (such as glycine, betaine, aminopropionic acid); imidazoline surfactants; amine oxides; amidobetaines.

Mixtures of surfactants may be used. In such mixtures there may be individual components which are liquid, provided that the carrier material overall, is a solid.

Anionic surfactants are particularly preferred as carrier materials.

### Inorganic Carrier Materials:

The carrier material can also be an water-soluble inorganic material which is neither a surfactant no a polymer. Simple organic salts have been found suitable, particularly in admixture with polymeric and/or surfactant carrier materials as described above. Suitable salts include carbonate, bicarbonates, halides, sulphates, nitrates and acetates, particularly soluble salts of sodium, potassium and magnesium. Preferred materials include, sodium carbonate, sodium bicarbonate and sodium sulphate. These materials have the advantage that they are cheap and physiologically acceptable. They are also relatively inert as well as compatible with many materials found in household and pharmaceutical products. In general spray-dried samples containing an inorganic carrier material gave smaller particle sizes on re-dissolution than comparative freeze-dried samples.

Mixtures of carrier materials are advantageous. Preferred mixtures include combinations of inorganic salts and surfactants and polymers and surfactants. Where inorganic salts are present the carrier material typically comprise 10-50%wt surfactant and 90-50%wt of the inorganic salt.

### Second liquid phase:

The compositions of the invention comprise a volatile, second liquid phase, which is not miscible with the aqueous solvent.

The second liquid phase of the emulsion may be selected from one or more from the following group of volatile organic solvents:
- alkanes, such as heptane, n-hexane, isooctane, dodecane, decane;
- cyclic hydrocarbons, such as toluene, xylene, cyclohexane;
- halogenated alkanes ,such as dichloromethane, dichoroethane, trichloromethane (chloroform), fluorotrichloromethane and tetrachloroethane;
- esters such as ethyl acetate;
- ketones such as 2-butanone;
- ethers such as diethyl ether;
- volatile cyclic silicones such as either linear or cyclomethicones containing from 4 to 6 silicon units. Suitable examples include DC245 and DC345, both of which are available from Dow Corning Inc.

Preferred second liquid phases have a boiling point of less than 150 Celsius and, more preferably, have a boiling point of less than 100 Celsius, so as to facilitate drying, particularly spray-drying under practical conditions and without use of specialised equipment. Preferred second liquid phase material are non-flammable, or have a flash point above the temperatures encountered in the method of the invention.

Preferably, the second liquid phase comprises from about 10 % to about 95 % v/v of the emulsion, more preferably from about 20 % to about 80 % v/v.

Particularly preferred solvents are halogenated solvents, more preferably chlorine-containing solvents, most preferably solvents selected from (di- or tri-chloromethane).

### Materials as carriers:

The products of the present invention include 'payload' materials to be dispersed when the products are partially dissolved in an aqueous medium. These are water-insoluble materials, which are soluble in the second liquid phase. Dispersion into an aqueous medium of such hydrophobic materials is much improved in the products of the invention. These hydrophobic materials are dissolved in the discontinuous phase of the emulsion prior to drying.

Solutions or dispersions comprising the carrier (surfactant and/or polymer) and a hydrophobic material can be formed by exposing to an aqueous medium products according to the present invention, wherein said products comprise the hydrophobic material and are obtainable by means of the method of the present invention.

The present invention is susceptible of a very broad range of applications as regards the choice of hydrophobic material to be carried. The embodiments mentioned below are selected from a range of applications including, home and personal care products, agrochemicals and pharmaceuticals, perfumes and flavourings, inks and dyes and other applications. The list is not intended to be limiting.

For example, there are many instances in personal care products such as deodorants, skin and hair cleaning or care products or in household products such as laundry cleaning and care products or household cleaning or care products for hard and soft surfaces where it is desirable to administer hydrophobic materials in an aqueous environment. Because of the hydrophobic nature of these materials they are often difficult to disperse in an aqueous environment. The use of the products of the present invention facilitates this dispersion and in many cases enables hydrophobic materials to be dispersed more effectively than previously.

It may be required to disperse the hydrophobic materials at the point where a formulation is being used. In this case the materials of the present invention will be contained in a formulation until it is used by exposing it to an aqueous environment, at which time the water-soluble/dispersible carrier forming the product will release the hydrophobic material.

Particularly preferred products according to the present invention comprise a water-insoluble, pharmaceutically active substance and a water-soluble physiologically acceptable carrier.

As well as finding advantageous application at the point of use, the products of the present invention may be used to introduce hydrophobic materials into formulations, for example, liquid formulations during the manufacture of these formulations. In this case the products of the present invention will, on contact an aqueous environment, release the hydrophobic material in a form in which it can be more readily incorporated into the product being manufactured.

The products of the present invention may be used to transport materials to sites where they can be incorporated into formulations. By converting liquids into products according to the invention, the need to transport large amounts of liquids can be avoided resulting in significant cost savings and safer transport of materials, which are potentially hazardous when transported in a liquid form. Materials which would be potentially unstable if stored or transported in liquid form may be incorporated into the products of the present invention and stored or transported with less risk of degradation.

It is envisaged that incorporation of potentially unstable hydrophobic materials, for example vaccines, vitamins or perfume components, into the products of the present invention will protect them from degradation during storage prior to use. For example, a so-called pro-fragrance incorporated into a dry carrier material according to the invention is expected to exhibit reduced hydrolysis as compared with the same material in solution or exposed to moist air.

Some specific examples of products in which the products of the present invention may be used are given below. These are given as examples only and are not intended to limit the applicability of the present invention. Those skilled in the art will however realise that the materials of the present invention will have utility in other areas not specifically exemplified herein.

Hydrophobic materials that are released from the products of the present invention at the time of use may include:-
- antimicrobial agents, for example: Triclosan™, climbazole, octapyrox, ketoconizole, phthalimoperoxyhexanoic acid (PAP), quaternary ammonium compounds;
- antidandruff agents for example: zinc pyrithione;
- skin lightening agents for example 4-ethylresorcinol;
- fluorescing agents for example: 2,5-bis(2-benzoxazolyl) thiophene for use on fabrics (such as cotton, nylon, polycotton or polyester)in laundry products;
- skin conditioning agents, for example cholesterol;
- antifoaming agents for example isoparrafin
- hair conditioning agents for example quaternary ammonium compounds, protein hydrolysates, peptides, ceramides and hydrophobic conditioning oils for example hydrocarbon oils such as paraffin oils and/or mineral oils, fatty esters such as mono-, di-, and triglycerides, silicone oils such as polydimethylsiloxanes (e.g. dimethicone) and mixtures thereof;
- fabric conditioning agents for example quaternary ammonium compounds having 1 to 3, preferably 2 optionally substituted (C8-C24) alk(en)yl chains attached to the nitrogen atom by one or more ester groups; hydrophobic monoparticles such as a sucrose polyester for example sucrose tetra-tallowate; silicones for example polydimethylsiloxane;
- thickening agents for example hydrophobically modified cellulose ethers such as modified hydroxyethylcelluloses;
- dyes for example dyes intended to change the colour of fabrics, fibres, skin or hair;
- UV protecting agents such as sunscreens for example octyl methoxycinnamate (Parsol MCX), butyl methoxydibenzoylmethane (Parsol 1789) and benzophenone-3 (Uvinul M-40), ferulic acid;
- bleach or bleach precursors for example 6-N-phthalimidoperoxyhexanoic acid (PAP) or photobleaching compounds. Dispersing the bleach from the porous bodies of the present invention results in the bleach being more finely dispersed and reduces the spot damage seen when larger particles of the bleach contact a fabric;
- antioxidants for example hydrophobic vitamins such as vitamin E, retinol, antioxiants based on hydroxytoluene such as Irganox™ or commercially available antioxidants such as the Trollox™ series.
- insecticides, pesticides, herbicides and other agrochemicals, for example those that are stored as solid compositions before use but which are made up into liquid for spraying (or other application) onto animals or crops;
- perfumes or flavourings or precursors thereto;
- pharmaceutically or veterinary active materials.

Compositions of the present invention may have additional specific advantages. For example, there is a need for pharmaceutical compositions, which can be taken by the consumer without the need to ingest the composition with a drink such as water. These compositions interact with the moisture in the oral cavity to release the active ingredient, which is then ingested by the consumer. By incorporating the pharmaceutically or veterinary active molecule in the products of the present invention, pharmaceutical compositions which meet this need can be prepared. In a similar way to that described above pharmaceutical and veterinary active ingredients may be formulated so that they release the active material into the nasal, ocular, pulmonary or rectal cavities or on the skin where they may act topically or they may be absorbed transdermally to act systemically.

By using the appropriate polymeric material in the products of the present invention, materials can be made that remain intact until the conditions (for example temperature or pH) change to those under which dispersion can occur. Thus dispersion can be delayed until a certain temperature has been reached or until the pH has changed to a suitable value such as would occur as the products pass down the GI tract.

Acidity in the GI tract reduces down the GI tract and materials which disperse hydrophobic actives only when the materials are exposed to higher pH conditions enable pharmaceutically or veterinary active materials to be released only in the intestine having passed through the stomach intact.

Further examples of situations where the products of the present invention are used to incorporate a hydrophobic material into a formulation during the manufacture of that product include:-
- the introduction of hydrophobic materials such as fluorescers; enzymes; bleaches; hydrophobic polymers for example hydrophobically modified polyacrylates, silicones, hydrophobically modified polyvinylpyrrolidone, sulpha alkyl polysaccharides, Jaguar and JR polymers; fatty alcohols or acids; dyes for example shading dyes or black dyes for colour recovery into laundry products;
- the use of products according to the present invention containing hydrophobic dyes in the manufacture of water soluble inkjet compositions;
- the introduction of products containing different hydrophobic materials enables a manufacturer to produce a single base formulation into which the desired hydrophobic materials may be introduced by the use of the appropriate product of the present invention;
- the use of products containing hydrophobic polymers which disperse into water to form a latex. The use of such latexes containing appropriate hydrophobic polymers deposited onto fabric imparts crease resistance or easy-iron properties to fabric.

The materials of the present invention may include within carrier material, water-soluble materials, which will be dispersed when the bodies are dispersed in an aqueous medium. The water-soluble materials may be incorporated into the lattice by dissolving them in the liquid medium from which they are made.

### Examples of suitable water soluble materials include:-

- Water soluble vitamins such as vitamin C;
- water soluble fluorescers such as the 4,4'-bis(sulfostyryl)biphenyl disodium salt (sold under the trade name Tinopal CBS-X;
- activated aluminium chlorohydrate;
- transition metal complexes used as bleaching catalysts;
- water soluble polymers and oligomers such as polyesters isophthalic acid), gerol, xanthan gum, or polyacrylates; diethylenetriamine-pentaacetic acid (DTPA);
or mixtures thereof

In some cases the water-soluble carrier material itself may not be inert but may also have useful activity. For example, the carriers suggested above include materials which are known to be active as detergency builders (sodium carbonate) or as an antacid (sodium bicarbonate).

### Method of Preparation:

As noted above, the method for preparing the products of the invention comprises spray-drying an emulsion containing an aqueous phase comprising a carrier and a volatile 'oil' phase comprising a 'payload' material.

Preferably, the volatile oil phase comprises from about 10 % to about 95 % v/v of the emulsion, more preferably from about 20 % to about 68 % v/v.

The emulsions are typically prepared under conditions which are well known to those skilled in the art, for example, by using a magnetic stirring bar, a homogeniser, or a rotational mechanical stirrer. The emulsions need not be particularly stable, provided that they do not undergo extensive phase separation prior to the drying step.

Homogenisation using a high-shear mixing device is a particularly preferred way to make an emulsion in which the aqueous phase is' the continuous phase. It is believed that this avoidance of coarse emulsion and reduction of the droplet size of the dispersed phase of the emulsion, results in an improved dispersion of the 'payload' material in the
dry product. In a preferred method according to the invention a water-continuous emulsion is prepared with an average dispersed-phase droplet size (using the Malvern peak intensity) of between 500nm and 3000nm. We have found that an 'Ultra-Turrux' T25 type laboratory homogeniser (or equivalent) gives a suitable emulsion when operated for more than a minute at above 10,000 rpm.

In preferred embodiments of the invention, there is a directional relation between the emulsion droplet size and the size of the particles of the 'payload' material, which can be detected after dispersion of the materials of the invention in aqueous solution. We have determined that an increase in the speed of homogenisation for precursor emulsions can decrease final particle size after re-dissolution.

It is believed that the re-dissolved particle size can be reduced by nearly one half when the homogenisation speed increased from 13,500 rpm to 21,500 rpm. The homogenisation time also plays an important role in controlling re-dissolved particle size. The particle size again decreases when increase the homogenisation time, and the particle size distribution becomes broader at the same time.

For an intermediate emulsion size range of 500-3000nm the re-dispersed particle size range is typically 180-300nm.

Spray drying is well known to those versed in the art. In the case of the present invention some care must be taken due to
the presence of a volatile non-aqueous solvent in the emulsion being dried. In order to reduce the risk of explosion when a flammable solvent is being used, an inert gas, for example nitrogen, can be employed as the drying medium in a so-called closed spray-drying system. The solvent can be recovered a re-used.

We have found that the 'Buchi' B-290 type laboratory spray drying apparatus is suitable.

The product form obtained form the spray drying process is a powder. As noted above, the precise microstructure of this powder is unclear. This powder can be incorporated into tablets, either by simple compression (where the carrier material is sticky enough) or following the addition of excipients and or tableting agents.

The present invention will now be more particularly described, by way of example only, with reference to the accompanying Examples.

### Examples:

### Particle sizing

A method of particle sizing for the dispersed products of the present invention used in the following examples employs a dynamic light scattering instrument (Nano S, manufactured by Malvern Instruments UK). Transmission electron microscope images and field flow fractionation techniques have been
used to confirm some particle sizing data. Specifically, the Malvern Instruments Nano S uses a red (633nm) 4mW Helium-Neon laser to illuminate a standard optical quality UV curvette containing a suspension of material. The viscosity of the sample can be measured with a Viscolite 700 portable viscometer (manufactured by Hydramotion UK) prior to analysis with the Malvern Instruments Nano S.

### Example 1:

In this example the emulsion that was spray dried comprised the water insoluble dye 'Sudan Red 7b' (CI 26050) and the water-soluble structuring agents are polyvinyl alcohol (PVA) and sodium dodecyl sulphonate (SDS). The emulsion was made up as shown in the table below:

**Table 2**

| **Sudan Red 7b** | **PVA** | **SDS** | **Cyclohexane** | **DI Water** |
|---|---|---|---|---|
| 0.1 g | 2.0 g | 5.0 g | 120ml | 40ml |

PVA was dissolved in 40ml water with stirring and once the entire polymer was dissolved the SDS was added. The red dye/cyclohexane solution was made up separately and added to the aqueous solution slowly over 10 minutes. Once all the dye was been added, stirring was continued for a further 5 minutes. Stirring and emulsification was achieved by means of a magnetic stir bar and stirrer.

The emulsion was spray dried using a Buchi™ laboratory spray dryer (fitted with a Schlick™ nozzle) housed in a laboratory fume cupboard. The emulsion was stirred continuously using a magnetic stirrer whilst spray drying. The following spray drying conditions were employed:

| | |
|---|---|
| Inlet temperature | 120°C |
| Atomisation pressure | 3.0 bar |
| Liquid feed rate | 3.0mL/Min |
| Outlet temperature (start) | 60°C |
| filter bag pressure (start) | -40mbar |

### Example 2:

In this example the emulsion that was spray dried comprised the water-insoluble antimicrobial 'Triclosan'™ and the water-soluble structuring agent polyvinyl alcohol. The emulsion was made up as shown in the table below:

**Table 3**

| **Triclosan** | **PVA** | **Cyclohexane** | **DI Water** |
|---|---|---|---|
| 8.13 | 54.4g | 563ml | 1875ml |

The PVA was dissolved in the deionised water. The Triclosan was dissolved in the cyclohexane. The Triclosan solution was added to the PVA solution over a period of 1 minute with continuous homogenisation (using an IKA ultra-Turrax™ T25 homogeniser at 6000rpm) and then further homogenised for a further 2 minutes (total of 3 minutes).

The emulsion was spray dried using a Buchi laboratory spray dryer (fitted with a Schlick nozzle) housed in a laboratory fume cupboard. The emulsion was stirred continuously using a magnetic stirrer whilst spray drying. The following spray drying conditions were employed:

| | |
|---|---|
| Inlet temperature | 90°C |
| Atomisation pressure | 4.0 bar |
| Liquid feed rate | 3.0mL/Min |
| Outlet temperature (start) | 60°C |
| filter bag pressure (start) | -40mbar |

31.7 grams of material were collected. In this case a hazy dispersion is formed on addition of spray dried material to water. After several hours an optically clear and colourless dispersion is obtained.

### Example 3:

In this example the emulsion that was spray dried comprised the water insoluble antimicrobial 'Triclosan'™ and the water soluble structuring agents polyvinyl alcohol and Brij 78™. The emulsion was made up as shown in the table below:

**Table 4**

| **Triclosan** | **Brij 78** | **PVA** | **Cyclohexane** | **DI Water** |
|---|---|---|---|---|
| 6.0g | 15.4g | 38.5g | 78ml | 770ml |

The PVA was dissolved in the de-ionised water. When the PVA was dissolved the Brij-78 was added and allowed to dissolve. Triclosan was dissolved in the cyclohexane. The Triclosan solution was added (over 30 seconds) to the solution of PVA/Brij 78 with continuous homogenisation (using an IKA ultra-Turrax T25 homogeniser at 6000rpm). The emulsion was further homogenised for a further 30 seconds (total of one minute).

The emulsion was spray dried using a Buchi laboratory spray dryer (fitted with a Schlick nozzle) housed in a laboratory fume cupboard. The emulsion was stirred continuously using a magnetic stirrer whilst spray drying. The following spray drying conditions were employed:

| | |
|---|---|
| Inlet temperature | 85°C |
| Atomisation pressure | 4.0 bar |
| Liquid feed rate | 3.0mL/Min |
| Outlet temperature (start) | 55°C |
| filter bag pressure (start) | -40mbar |

32.4 grams of material was collected.

### Examples 4-13:

In this example the emulsion that was spray dried comprised the water insoluble antimicrobial 'Triclosan'™ and the water soluble structuring agent SDS. A range of solvents, as listed below was employed.

**Table 5**

| **Example** | **Solvent** | **Boiling Point** | **Solvent Type** |
|---|---|---|---|
| **4** | Pentane | 36°C | Alkane |
| **5** | Hexane | 69°C | Alkane |
| **6** | Heptane | 98°C | Alkane |
| **7** | Octane | 125°C | Alkane |
| **8** | Nonane | ∼150°C | Alkane |
| **9** | Chloroform | 61°C | Chlorinated solvent |
| **10** | t-Butylmethyl ether | 55°C | Ethers |
| **11** | Cyclohexane | 81°C | Cyclic solvents |
| **12** | Ethyl Acetate | 77°C | Ester |
| **13** | Toluene | 110°C | Aromatic solvents |

The following solutions were made:

| | |
|---|---|
| SDS | 8.9g dissolved in 125ml water |
| Triclosan | 1.1g dissolved in 125ml organic solvent |

The Triclosan solution was slowly added to the SDS solution over a 30 second period, with continuous homogenisation using a IKA ultra-Turrax T25 homogeniser (6,000rpm). After addition, homogenisation is continued for a further 90 seconds (2 minutes in total).

Each emulsion was spray dried using a Buchi laboratory spray dryer (fitted with a Schlick nozzle) housed in a laboratory fume cupboard. During spray drying the emulsion was stirred continuously using a magnetic stirrer. The following spray drying conditions were used:

| | |
|---|---|
| Inlet temperature | 90°C |
| Atomisation pressure | 3.0bar |
| Liquid feed rate | 3.0mL/min |
| Outlet temperature (start) | Varies with solvent |
| filter bag pressure (start) | -40mbar |

### Example 14:

In this example the emulsion that was spray dried comprised the water insoluble fluorescer 'Tinopal SOP'™ and the water-soluble structuring agent CocoPAS (sodium lauryl sulphate, EMAL 10PHD, ex Kao). The emulsion was made up as shown in the table below:

**Table 6**

| **Tinopal SOP** | **Cocopas** | **Dichloromethane** | **DI Water** |
|---|---|---|---|
| 1.0g | 9.0g | 120ml | 120ml |

The CocoPAS was dissolved in the de-ionised water. The Tinopal SOP was dissolved in the dichloromethane.

The dichloromethane solution was added (over 30 seconds) to the aqueous solution of cocoPAS with continuous homogenisation (using an IKA ultra-Turrax T25 homogeniser at 6000rpm) for a total of 1 minute.

The resulting emulsion was spray dried using a Buchi laboratory spray dryer (fitted with a Schlick nozzle) housed in a laboratory fume cupboard. The emulsion was stirred continuously using a magnetic stirrer whilst spray drying. The following spray drying conditions were employed:

| | |
|---|---|
| Inlet temperature | 120°C |
| Atomisation pressure | 3.0 bar |
| Liquid feed rate | 4.0mL/Min |
| Outlet temperature (start) | 66°C |
| filter bag pressure (start) | -40mbar |

3.84g of material was collected

### Example 15:

In order to compare the effectiveness of compositions of the invention with those prepared by freeze-dry methods, the performance of the product obtained in example 14 was compared with a product obtained by a freeze drying method.

To prepare the freeze dried product, 9g of CocoPAS (sodium lauryl sulphate, EMAL 10PHD, ex Kao) was dissolved in 120 ml of water. To this aqueous solution was added 1.0g of Tinopal™ SOP in 120ml of dichloromethane with vigorous stirring. The emulsion formed was sprayed into liquid nitrogen using a trigger spray and the resulting frozen powder was freeze-dried (Edwards Supermodulyo, operated with an average vacuum of 0.2mbar and at -50 °C) to form a powder.

Samples of the 10% loaded product were added to Brilhante™ (ex Gessy Lever, 2004) base formulation, without any fluorescer present. The composition thus obtained was used to launder untreated cloth monitors. The change in Ganz whiteness (Delta G) on cloth monitors was measured after each wash. Measurements were performed for cotton, nylon and polyester. Results for the method of the invention (in bold, and marked 'spray dried') and the comparative tests (marked 'freeze dried') are shown in the table below:

**Table 7**

| **Cloth Type** | **Preparation** | **Ganz after 1^{st} wash** | **Ganz after 2^{nd} wash** | **Ganz after 3^{rd} wash** |
|---|---|---|---|---|
| Nylon | Freeze dried | 33.35 | 46.84 | 53.14 |
| **Nylon** | **Spray dried** | **38.17** | **50.05** | **56.14** |
| Polyester | Freeze dried | 9.11 | 12.46 | 14.74 |
| **Polyester** | **Spray dried** | **12.05** | **15.23** | **16.73** |
| Cotton | Freeze dried | 21.68 | 26.43 | 30.36 |
| **Cotton** | **Spray dried** | **25.39** | **32.64** | **34.71** |

Higher 'Ganz' figures are obtained when fluorescer is more effectively deposited. Products prepared according to the method of the invention show better performance than those prepared by the freeze-drying method.

### Example 16:

In order to demonstrate the effect of choice of water soluble polymers on particle size and distributions, several different water soluble polymers with different molecular weights, including polyvinyl alcohol (PVA), hydroxypropyl methyl cellulose (HPMC), hydroxyethyl cellulose (HEC), and polyvinylpyrrolidone (PVP) were employed. Table 8 lists the polymers.

**Table 8**

| **Polymer** | **Molecular weight, mol/l** | **Producer** |
|---|---|---|
| PVA | Mw 9,000-10,000 | Aldrich |
| HPMC | Mw 10,000 | Aldrich |
| HEC | Mw 90,000 | Aldrich |
| PVP | Mw 10,000 | Aldrich |
| | Mw 24,000 | Fluka |
| | Mw 55,000 | Fluka |
| | Mw 360,000 | Fluka |

In this example, Oil red O (OR) was used as a model for active compound, and sodium dodecylsulfate (SDS) was used as a surfactant carrier.

0.25 g OR was dissolved in 50 ml chloroform as oil phase, and 1.0 g SDS and 1.0 g PVP were dissolved in 50 ml water as aqueous phase. The oil phase was added dropwise into aqueous phase with overhead stirring (IKA EUROSTAR) at 600 rpm for 2 min.

The coarse emulsion was then further treated with a homogenizer (IKA T25 basic Ultra-Turrax) at 17,500 rpm for 5 min. The resulting fine emulsion was then spray dried at 125 °C with a Buchi Mini spray dryer B-290 to obtain products according to the present invention. Spray drying conditions were Inlet temp.: 125 °C; Aspiration rate: 100%; Pump rate: 3.62ml/min.

A sample of the dry powder was then dispersed into distilled water and the nanoparticle size was measured with Malvern Nano-S. Details concerning these experiments and their results are summarized in Table 9.
PhR=phase ratio, oil phase/aqueous phase
PS=average particle size
PDI=polydispersity index

**Table 9**

| **Sample** | **Oil Phase** | **Aqueous phase** | | | **PhR, v/v** | **PS, nm** | **PDI** |
|---|---|---|---|---|---|---|---|
| | **OR, mg/ml** | **SDS, mg/ml** | **PVP, mg/ml** | | | | |
| WP-141 | 5.0 | 20.0 | Mw 10,000 | 20.0 | 50/50 | 255 | 1.016 |
| WP-142 | | | Mw 29,000 | | | 220 | 1.103 |
| WP-143 | | | Mw 55,000 | | | 255 | 1.133 |
| WP-144 | | | Mw 360,000 | | | 295 | 1.125 |

In further examples 0.25 g OR was dissolved in 50 ml chloroform as oil phase, and 1.0 g SDS and 1.0 g water soluble polymer (PVP (Mw 29,000), PVA, HPMC, or HEC) were dissolved in 50 ml water as aqueous phase. The oil phase was added dropwise into aqueous phase with overhead stirring at 600 rpm for 2 min. The coarse emulsion was further treated with a homogenizer at 17,500 rpm for 5 min. The resulting fine emulsion was then spray dried at 125 °C with a Buchi Mini spray dryer B-290 (Inlet temp.: 125 °C; Aspiration rate: 100%; Pump rate: 3.62ml/min.)

A sample of the dry powder was then dispersed into distilled water and the nanoparticle size was measured with Malvern Nano-S. Details concerning these experiments and their results are summarized in Table 10.

**Table 10**

| **Sample** | **Oil Phase** | **Aqueous phase** | | | **PhR, v/v** | **PS, nm** | **PDI** |
|---|---|---|---|---|---|---|---|
| | **OR, mg/ml** | **SDS, mg/ml** | **Water soluble polymer, mg/ml** | | | | |
| WP-142 | 5.0 | 20.0 | PVP | 20.0 | 50/50 | 220 | 1.103 |
| WP-145 | | | PVA | | | 220 | 1.240 |
| WP-146 | | | HPMC | | | 164 | 1.012 |
| WP-147 | | | HEC | | | 342 | 1.567 |

In this example HPMC (Mw 10,000) gave the smallest particle size and narrowest poly-dispersity, PVA (9-10k MW) gave similar particle size to PVP but broader particle size distributions, and HEC (MW 90k) gave the largest particle size and the broadest particle size distributions.

In yet further examples 0.25 g OR was dissolved in 50 ml chloroform as oil phase, and 1.0 g SDS was dissolved in 50 ml water with different water soluble polymers (PVP (Mw 29,000), PVA (Mw 10,000), and HPMC (Mw 10,000)) with different concentrations (20mg/ml, 15mg/ml, 10mg/ml, and 5mg/ml) as aqueous phase. The oil phase was added dropwise into the aqueous phase with overhead stirring at 600 rpm for 2 min. The coarse emulsion was further treated with a homogenizer at 17,500 rpm for 5 min.

The resulting fine emulsion was then spray dried at 125 °C with a Buchi Mini spray dryer B-290 (Inlet temp.: 125 °C; Aspiration rate: 100%; Pump rate: 3.62ml/min).

A sample of the dry powder was then dispersed into distilled water and the dispersed particle size was measured with Malvern Nano-S. Details concerning these experiments and their results are summarized in Table 11.

**Table 11**

| **Sample** | **Oil Phase** | **Aqueous phase** | | | **PhR, v/v** | **PS, nm** | **PDI** |
|---|---|---|---|---|---|---|---|
| | **OR, mg/ml** | **SDS, mg/ml** | **Water soluble polymer, mg/ml** | | | | |
| WP-142 | 5.0 | 20.0 | PVP | 20.0 | 50/50 | 220 | 1.103 |
| WP-145 | | | PVA | | | 220 | 1.240 |
| WP-146 | | | HPMC | | | 164 | 1.012 |
| WP-148 | | | PVP | 15.0 | | 295 | 1.026 |
| WP-149 | | | PVA | | | 255 | 1.027 |
| WP-150 | | | HPMC | | | 164 | 1.067 |
| WP-151 | | | PVP | 10.0 | | 585 | 1.309 |
| WP-152 | | | PVA | | | 255 | 1.137 |
| WP-153 | | | HPMC | | | 220 | 1.180 |
| WP-154 | | | PVP | 5.0 | | 615 | 1.314 |
| WP-155 | | | PVA | | | 342 | 1.230 |
| WP-156 | | | HPMC | | | 396 | 1.764 |

### Example 17:

0.25 g OR was dissolved in 60 ml chloroform as oil phase, and 4.05 g SDS and 7.0 g PVA (Mw 10,000) were dissolved in 140 ml water as aqueous phase. The oil phase was added dropwise into aqueous phase with overhead stirring at 600 rpm for 20 min. Half the volume of the coarse emulsion obtained was further treated with a homogenizer at 17,500 rpm for 5 min.

The coarse emulsion and the fine emulsion were then each spray dried at 120 °C with a Buchi Mini spray dryer B-290 respectively (inlet temp.: 120 °C; Aspiration rate: 100%; Pump rate: 5.90 ml/min.). Samples of the dry powders were dispersed into distilled water and the particle size was measured with Malvern Nano-S. Details concerning these experiments and their results are summarised in Table 12.

In a separate preparation 0.20 g OR was dissolved in 50 ml chloroform as oil phase, and 1.45 g SDS and 2.50 g PVA (Mw 10,000) were dissolved in 50 ml water as aqueous phase. The oil phase was added dropwise into aqueous phase with overhead stirring at 600 rpm for 2 min. The coarse emulsion was further treated with a homogeniser at 17,500 rpm for 5 min.

This fine emulsion was then spray dried at 120 °C with a Buchi Mini spray dryer B-290 (inlet temp.: 120 °C; Aspiration rate: 100%; Pump rate: 5.90 ml/min.). The dry powder was then dispersed into distilled water and the particle size was measured with Malvern Nano-S. Details concerning these experiments and their results are also summarised in Table 12.

**Table 12**

| **Sample** | **Oil Phase** | **Aqueous phase** | | **PhR, v/v** | **Process** | **PS, nm** | **PDI** |
|---|---|---|---|---|---|---|---|
| | **OR, mg/ml** | **SDS, mg/m l** | **PVA, mg/ml** | | | | |
| WP-101 | 4.0 | 29.0 | 50.0 | 30/70 | Stir | 264 | 1.112 |
| WP-102 | | | | | Homogenise | 89 | 1.012 |
| WP-103 | | | | 50/50 | | 123 | 1.019 |

### Example 18:

0.25 g OR was dissolved in 50 ml chloroform as oil phase, and 1.0 g SDS and 1.0 g PVP (Mw 29,000) were dissolved in 50 ml water as aqueous phase. The oil phase was added dropwise into the aqueous phase with overhead stirring at 600 rpm for 2 min.

The coarse emulsion was further treated with a homogeniser at different speed to obtain a series of finer emulsions. These finer emulsions were then spray dried at 125 °C with a Buchi Mini spray dryer B-290 (inlet temp.: 125 °C; Aspiration rate: 100%; Pump rate: 3.62 ml/min).

The dry powder was then dispersed into distilled water and the nanoparticle size was measured with Malvern Nano-S. Details concerning these experiments and their results are summarized in Table 13 (HS=Homogenization speed, Ht=Homogenization time)

**Table 13**

| **Sample** | **Oil Phase** | **Aqueous phase** | | **PhR, v/v** | **HS, rpm** | **Ht, min** | **PS, nm** | **PDI** |
|---|---|---|---|---|---|---|---|---|
| | **OR, mg/ml** | **SDS, mg/ml** | **PVA, mg/ml** | | | | | |
| WP-157 | 4.0 | 29.0 | 50.0 | 50/50 | 13,500 | 2 | 347 | 1.037 |
| WP-158 | | | | | 17,500 | 2 | 279 | 1.043 |
| WP-142 | | | | | 17,500 | 5 | 220 | 1.103 |
| WP-159 | | | | | 21,500 | 2 | 190 | 1.014 |

### Example 19:

Preparations have been made into tablet form by means of a tablet maker which has four parts. Three used to form the tablet and one to achieve compression. The parts used for tablet formation are an inverted "U" shape with a hole in the top, into which fits a solid cylinder with arms at the top and a solid rectangle that fits into the inverted U. Tablets are formed, with the U and rectangle assembled, by adding powder to the hole in the inverted U and carefully placing the cylinder into the hole. The assembly is then placed in the compression part, where the screw thread acts on the cylinder compacting the powder. The tablet is released by removing the rectangular and cylindrical blocks.

Spray dried powder prepared from cocoPAS, sodium sulphate and fat red dye was made into a 0.5g tablet using this apparatus. When a similar freeze dried powder was used, (cocoPAS, sodium chloride, fat red dye), a 0.06g tablet was formed.

Tablet and powder behave differently in terms of dissolution times and clarity of solution.

### Example 20:

A product comprising sodium sulphate was made by dissolving 0.5g CocoPAS in 12 ml water and adding 0.5g sodium sulphate. 0.01g Fat red 7B dye was dissolved in cyclohexane, added to the aqueous solution to form an emulsion and freeze dried as a powder. Other similar powders, varying the CocoPAS : Sodium sulphate ratio from 60:40 to 90:10 gradually, were made in the same way. A similar set of samples containing sodium carbonate was also made. All powders re-dissolved in water to give a clear solution. Without sodium carbonate, dissolution took about 20 seconds, with sodium carbonate, the time increased to 1 to 2 minutes.

A CocoPAS:NaSO4, 50:50 ratio powder was made, this time forming the emulsion using a homogeniser and spray drying; for comparison of particle size, which gave a Z (Malvern) average of 57.3 and a poly-dispersity of 0.39. For a 20:80 cocoPAS:Na2SO4 sample the particle size Z (Malvern) average was 115 and a poly-dispersity of 0.126.

For freeze dried samples, the particle size would appear to decrease as the amount of filler increases, for sodium sulphate, range Z ave. 2000, polydispersity 0.746 (50:50) to Z ave. 609, polydispersity 0.492 (10:90).

Other inorganic fillers included sodium carbonate, sodium chloride, magnesium chloride, magnesium acetate, calcium chloride and sucrose; these tended not to give clear solutions. Whereas sodium bicarbonate, sodium acetate and magnesium sulphate (at 50:50) gave clear re-dispersions. All these were prepared in a PVA/SDS system. Particle sizes varied between Z ave. 1380, polydispersity 0.6 and Z ave. 771, polydispersity 0.48 for the sodium salt fillers.

The preparations containing magnesium salts at 50:50 in PVA/SDS, gave particle sizes that ranged between Z ave. 72.9, polydispersity 0.402 and Z ave. 672, polydispersity 0.655. Magnesium sulphate was also made at the 20:80 ratio, but unlike sodium sulphate, due to it not giving a clear solution, the particle size Z ave.(255) polydispersity 0.375 was greater than the 50:50 ratio Z ave. (72.9) polydispersity 0.402. The particle size data for magnesium salts, for all but the acetate, gave more than one peak size.

Calcium chloride and sucrose were also used as bulking agents; they gave particle sizes of Z ave.892, polydispersity 0.593 and Z ave.338, polydispersity 0.489 respectively.

Polymer only i.e. PVA with sodium sulphate filler (50:50) has been prepared as a freeze dried powder. It was slow to re-dissolve; time, 7 minutes, gave a clear solution and particle size Z ave. 467, polydispersity 0.557 and had 2 peaks.

### Example 21:

0.037 g of the hydrophobic polymer polycaprolactone was dissolved in 15 ml chloroform as oil phase, and 0.26 g SDS and 0.45 g PVA (Mw 10,000) were dissolved in 15 ml water as aqueous phase. The oil phase was added dropwise into the aqueous phase and homogenised for 2 min.

The emulsion was spray dried at 150 °C with a Buchi Mini spray dryer B-290 respectively (inlet temp.: 150 °C; Aspiration rate: 100%; Pump rate: 7.20 ml/min.). A sample of the dry powders were then dispersed into distilled water (10mg/ml) and the resulting particle size was measured with Malvern Nano-S. The particles were 162nm with a standard deviation of 2.14nm and a poly-dispersity of 0.131

### Example 22:

0.5 g of the hydrophobic polymer Eudragit™ EPO was dissolved in 60 ml chloroform as oil phase, and 10 g PVA (Mw 10,000) were dissolved in 120 ml water as aqueous phase. The oil phase was added dropwise into the aqueous phase and homogenised for 1 min.

The emulsion was spray dried at 90 °C with a Buchi Mini spray dryer B-290 respectively (inlet temp.: 90 °C; Aspiration rate: 100%; Pump rate: 3 ml/min.). A sample of the dry powders were then dispersed into distilled water (10mg/ml) and the resulting particle size was measured with Malvern Nano-S. The particles were 520nm.

## Claims

1. A method comprising the steps of:
(i) providing an emulsion of:
a) an aqueous solvent,
b) a carrier material soluble in (a), said carrier material being solid at ambient temperature,
c) a volatile second liquid phase which is not miscible with (a), and
d) a material which is soluble in (c) but not in (a), and,
(ii) spray-drying the emulsion at above 100 Celsius to simultaneously remove (a) and (c) and thereby obtain a powder, in which the carrier material (b) has material (d) dispersed therein, said dispersion of (d) being a nano-scale dispersion,
wherein the carrier material (b) is a polymer and/or a surfactant,
wherein the polymer is a water-soluble polymer selected from the group consisting of:
natural polymers;
cellulose derivatives;
homopolymers of any one of, or copolymers prepared from two or more of, the following monomers: vinyl alcohol, acrylic acid, methacrylic acid, acrylamide, methacrylamide, acrylamide methylpropane sulphonates, aminoalkylacrylates, aminoalkylmethacrylates, hydroxyethylacrylate, hydroxyethylmethylacrylate, vinyl pyrrolidone, vinyl imidazole, vinyl amines, vinyl pyridine, ethyleneglycol and other alkylene glycols, ethylene oxide and other alkylene oxides, ethyleneimine, styrenesulphonates, ethyleneglycolacrylates, ethyleneglycol methacrylate; and
mixtures thereof,
wherein the surfactant is non-ionic and/or anionic,
wherein the non-ionic surfactant is selected from the group consisting of:
ethoxylated triglycerides, fatty alcohol ethoxylates, alkylphenol ethoxylates, fatty acid ethoxylates, fatty amide ethoxylates, fatty amine ethoxylates, sorbitan alkanoates, ethylated sorbitan alkanoates, alkyl ethoxylates, Pluronics™, alkyl polyglucosides, stearol ethoxylates, alkyl polyglycosides, and mixtures thereof, and
wherein the anionic surfactant is selected from the group consisting of:
alkylether sulfates, alkylether carboxylates, alkylbenzene sulfonates, alkylether phosphates, dialkyl sulfosuccinates, alkyl sulfonates, soaps, alkyl sulfates, alkyl carboxylates, alkyl phosphates, paraffin sulfonates, secondary n-alkane sulfonates, alpha-olefin sulfonates, isethionate sulfonates, and mixtures thereof.

2. A method according to claim 1 wherein the aqueous solvent (a) is water.

3. A method according to claim 1 or 2 wherein the carrier material (b) is a polymer.

4. A method according to claim 3 wherein the carrier material is polyvinyl alcohol.

5. A method according to claim 1 or 2 wherein the carrier material (b) is a surfactant.

6. A method according to claim 5 wherein the carrier material (b) is an anionic surfactant.

7. A method according to any of claims 1 to 6 wherein the volatile second liquid phase (c) has a boiling point of less than 150 Celsius.

8. A method according to claim 7 wherein the volatile second liquid phase (c) has a boiling point of less than 100 Celsius.

9. A method according to claim 7 or claim 8 wherein the volatile second liquid phase (c) is selected from: alkanes, chlorinated solvents, ethers, cyclic solvents, esters and aromatic solvents.

10. A method according to any of claims 1 to 9 wherein the volatile second liquid phase comprises from 10 % to 95 % v/v of the emulsion.

11. A method according to claim 10 wherein the volatile second liquid phase comprises from 20 % to 68 % v/v.

## Patentansprüche

1. Verfahren, umfassend folgende Schritte:
(i) Bereitstellen einer Emulsion aus:
a) einem wässrigen Lösungsmittel,
b) einem in (a) löslichen Trägermaterial, wobei das Trägermaterial bei Umgebungstemperatur fest ist,
c) einer flüchtigen zweiten Flüssigphase, die mit (a) nicht mischbar ist, und
d) einem Material, das in (c) aber nicht in (a) löslich ist, und
(ii) Sprühtrocknen der Emulsion bei über 100 °C, um gleichzeitig (a) und (c) zu entfernen und dadurch ein Pulver zu erhalten, in welchem das Trägermaterial (b) das Material (d) darin dispergiert aufweist, wobei die Dispersion von (d) eine Dispersion im Nanobereich ist,
wobei das Trägermaterial (b) ein Polymer und/oder ein Tensid ist,
wobei das Polymer ein wasserlösliches Polymer ist, das ausgewählt ist aus der Gruppe bestehend aus:
natürlichen Polymeren;
Cellulosederivaten;
Homopolymeren eines beliebigen der folgenden oder Copolymeren aus zwei oder mehreren der folgenden Monomeren: Vinylalkohol, Acrylsäure, Methacrylsäure, Acrylamid, Methacrylamid, Acrylamidmethylpropansulfonaten, Aminoalkylacrylaten, Aminoalkylmethacrylaten, Hydroxyethylacrylat, Hydroxyethylmethylacrylat, Vinylpyrrolidon, Vinylimidazol, Vinylaminen, Vinylpyridin, Ethylenglykol und anderen Alkylenglykolen, Ethylenoxid und anderen Alkylenoxiden, Ethylenimin, Styrolsulfonaten, Ethylenglykolacrylaten, Ethylenglykolmethacrylat; und
Mischungen davon,
wobei das Tensid nichtionisch und/oder anionisch ist,
wobei das nichtionische Tensid ausgewählt ist aus der Gruppe bestehend aus: ethoxylierten Triglyceriden, Fettalkoholethoxylaten, Alkylphenolethoxylaten, Fettsäureethoxylaten, Fettamidethoxylaten, Fettaminethoxylaten, Sorbitanalkanoaten, ethylierten Sorbitanalkanoaten, Alkylethoxylaten, Pluronics™, Alkylpolyglucosiden, Stearolethoxylaten, Alkylpolyglykosiden und Mischungen davon, und
wobei das anionische Tensid ausgewählt ist aus der Gruppe bestehend aus:
Alkylethersulfaten, Alkylethercarboxylaten, Alkylbenzolsulfonaten, Alkyletherphosphaten, Dialkylsulfosuccinaten, Alkylsulfonaten, Seifen, Alkylsulfaten, Alkylcarboxylaten, Alkylphosphaten, Paraffinsulfonaten, sekundären n-Alkansulfonaten, α-Olefinsulfonaten, Isethionatsulfonaten und Mischungen davon.

2. Verfahren nach Anspruch 1, wobei das wässrige Lösungsmittel (a) Wasser ist.

3. Verfahren nach Anspruch 1 oder 2, wobei das Trägermaterial (b) ein Polymer ist.

4. Verfahren nach Anspruch 3, wobei das Trägermaterial Polyvinylalkohol ist.

5. Verfahren nach Anspruch 1 oder 2, wobei das Trägermaterial (b) ein Tensid ist.

6. Verfahren nach Anspruch 5, wobei das Trägermaterial (b) ein anionisches Tensid ist.

7. Verfahren nach einem der Ansprüche 1 bis 6, wobei die flüchtige zweite Flüssigphase (c) einen Siedepunkt von weniger als 150° Celsius hat.

8. Verfahren nach Anspruch 7, wobei die flüchtige zweite Flüssigphase (c) einen Siedepunkt von weniger als 100 Celsius.

9. Verfahren nach Anspruch 7 oder Anspruch 8, wobei die flüchtige zweite Flüssigphase (c) ausgewählt ist aus: Alkanen, chlorierten Lösungsmitteln, Ethern, zyklischen Lösungsmitteln, Estern und aromatischen Lösungsmitteln.

10. Verfahren nach einem der Ansprüche 1 bis 9, wobei die flüchtige zweite Flüssigphase von 10% bis 95% Volumen/Volumen der Emulsion ausmacht.

11. Verfahren nach Anspruch 10, wobei die flüchtige zweite Flüssigphase von 20% bis 68% Volumen/Volumen ausmacht.

## Revendications

1. Procédé comprenant les étapes consistant à:
(i) fournir une émulsion de:
a) un solvant aqueux,
b) un matériau support soluble dans (a), ledit matériau support étant solide à température ambiante,
c) une seconde phase liquide volatile qui n'est pas miscible avec (a), et
d) une matière qui est soluble dans (c) mais pas dans (a), et,
(ii) sécher par pulvérisation l'émulsion au-dessus de 100 °C pour éliminer simultanément (a) et (c) et obtenir ainsi une poudre, dans laquelle le matériau support (b) comporte la matière (d) dispersée dedans, ladite dispersion de (d) étant une dispersion à l'échelle nanométrique,
dans lequel le matériau support (b) est un polymère et/ou un tensioactif,
dans lequel le polymère est un polymère hydrosoluble choisi dans le groupe constitué par:
les polymères naturels;
les dérivés de cellulose;
les homopolymères de n'importe lequel parmi, ou les copolymères préparés à partir de deux ou plus parmi, les monomères suivants: alcool vinylique, acide acrylique, acide méthacrylique, acrylamide, méthacrylamide, méthylpropanesulfonates d'acrylamide, acrylates d'aminoalkyle, méthacrylates d'aminoalkyle, acrylate d'hydroxyéthyle, acrylate d'hydroxyéthylméthyle, vinylpyrrolidone, vinylimidazole, amines vinyliques, vinylpyridine, éthylèneglycol et autres alkylèneglycols, oxyde d'éthylène et autres oxydes d'alkylène, éthylèneimine, styrènesulfonates, acrylates d'éthylèneglycol, méthacrylate d'éthylèneglycol; et
les mélanges de ceux-ci,
dans lequel le tensioactif est non ionique et/ou anionique,
dans lequel le tensioactif non ionique est choisi dans le groupe constitué par:
les triglycérides éthoxylés, éthoxylates d'alcool gras, éthoxylates d'alkylphénol, éthoxylates d'acide gras, éthoxylates d'amide gras, éthoxylates d'amine grasse, alcanoates de sorbitane, alcanoates de sorbitane éthylés, éthoxylates d'alkyle, Pluronics™, polyglucosides d'alkyle, éthoxylates de stéarol, polyglycosides d'alkyle, et leurs mélanges, et
dans lequel le tensioactif anionique est choisi dans le groupe constitué par:
les sulfates d'alkyléther, carboxylates d'alkyléther, sulfonates d'alkylbenzène, phosphates d'alkyléther, sulfosuccinates de dialkyle, sulfonates d'alkyle, savons, sulfates d'alkyle, carboxylates d'alkyle, phosphates d'alkyle, paraffine-sulfonates, n-(alcane secondaire)sulfonates, alpha-oléfine-sulfonates, sulfonates d'iséthionate, et leurs mélanges.

2. Procédé selon la revendication 1 dans lequel le solvant aqueux (a) est l'eau.

3. Procédé selon la revendication 1 ou 2 dans lequel le matériau support (b) est un polymère.

4. Procédé selon la revendication 3 dans lequel le matériau support est un alcool polyvinylique.

5. Procédé selon la revendication 1 ou 2 dans lequel le matériau support (b) est un tensioactif.

6. Procédé selon la revendication 5 dans lequel le matériau support (b) est un tensioactif anionique.

7. Procédé selon n'importe lesquelles des revendications 1 à 6 dans lequel la seconde phase liquide volatile (c) a un point d'ébullition inférieur à 150 °C.

8. Procédé selon la revendication 7 dans lequel la seconde phase liquide volatile (c) a un point d'ébullition inférieur à 100 °C.

9. Procédé selon la revendication 7 ou la revendication 8 dans lequel la seconde phase liquide volatile (c) est choisie parmi: alcanes, solvants chlorés, éthers, solvants cycliques, esters, et solvants aromatiques.

10. Procédé selon n'importe lesquelles des revendications 1 à 9 dans lequel la seconde phase liquide volatile constitue 10% à 95% v/v de l'émulsion.

11. Procédé selon la revendication 10 dans lequel la seconde phase liquide volatile constitue 20% à 68% v/v.
